(19) Europäisches Patentamt
European Patent Office
Office européen des brevets

(11) EP 0 707 011 A1

(12) **EUROPEAN PATENT APPLICATION**
published in accordance with Art. 158(3) EPC

(43) Date of publication:
**17.04.1996 Bulletin 1996/16**

(21) Application number: **94918589.6**

(22) Date of filing: **28.06.1994**

(51) Int. Cl.[6]: **C07J 9/00**, A61K 31/575, A61K 31/56

(86) International application number: **PCT/JP94/01044**

(87) International publication number:
**WO 95/01367 (12.01.1995 Gazette 1995/03)**

(84) Designated Contracting States:
**AT BE CH DE DK ES FR GB GR IE IT LI LU MC NL PT SE**

(30) Priority: **30.06.1993 JP 159814/93**

(71) Applicant: **TAISHO PHARMACEUTICAL CO. LTD**
**Tokyo 171 (JP)**

(72) Inventors:
- **YAMADA, Yasuji**
**Tokyo 192-03 (JP)**
- **YAMAGISHI, Takehiro**
**Taisho Pharmaceutical Co., Ltd**
**Tokyo 171 (JP)**

(74) Representative: **Weisert, Annekäte, Dipl.-Ing. Dr.-Ing. et al**
**Patentanwälte**
**Kraus Weisert & Partner**
**Thomas-Wimmer-Ring 15**
**D-80539 München (DE)**

(54) **STEROL COMPOUND**

(57) A sterol compound represented by general formula (I), wherein X represents halogen; $R^1$ and $R^2$ represent each independently hydrogen or $C_2$-$C_5$ alkanoyl; and A represents -CO- or -CH(OH)-. The invention provides a novel sterol compound isolated from a sponge of the genus *Xestospongia* and another novel sterol compound by chemically modifying the above compound. The invention also provides aragusterol C and a novel sterol compound by chemically modifying the known aragusterol A. The obtained compounds are superior to aragusterol A in antineoplastic activity and useful as a medicine.

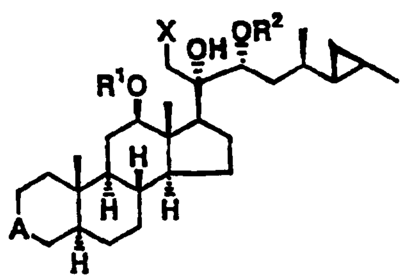

(I)

EP 0 707 011 A1

## Description

### Technical Field

The present invention relates to sterol compounds having an antitumor activity and being useful as medicines.

### Background Art

A compound described in Japanese Patent Laid-open No. 5-4998 (hereinafter referred to as "aragusterol A") is known as a compound similar in structure to the compounds of the present invention.

An object of the present invention is to provide novel sterol compounds having more excellent antitumor activity.

### Disclosure of the Invention

Sponges of the genus Xestospongia can be collected at coral reefs in the sea near Iriomote-jima, Okinawa, Japan. The present inventors have found that the extract of sponges of the genus Xestospongia contains a novel sterol compound having an antitumor activity, chemical modification of this compound gives novel compounds having an excellent antitumor activity, and chemical modification of known aragusterol A gives novel sterol compounds having an antitumor activity, and thereby the present invention has been accomplished.

The present invention is a sterol compound represented by the following formula:

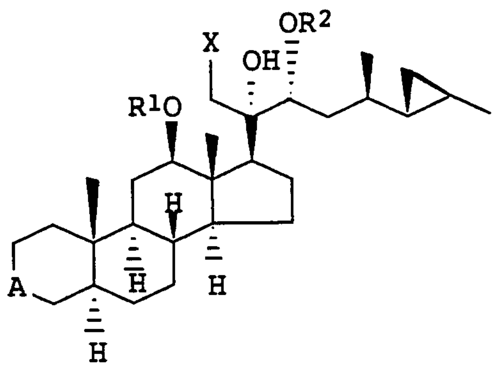

wherein X is a halogen atom, $R^1$ and $R^2$ are the same or different, and are each a hydrogen atom or an alkanoyl group having 2 to 5 carbon atoms, and A is -CO- or -CH(OH)-.

In the present invention, the halogen atom refers to a fluorine atom, a chlorine atom, a bromine atom and an iodine atom, and preferably a chlorine atom. The alkanoyl group refers to one which is straight or branched, for example, an acetyl group, a propionyl group or a butyryl group, and preferably an acetyl group.

The compounds of the present invention can be isolated or prepared by the following processes.

Firstly, a sterol compound of the present invention wherein $R^1 = R^2 = H$, X = Cl and A = -CO-(hereinafter referred to as "aragusterol C") can be isolated by extracting sponges of the genus Xestospongia with an organic solvent, followed by fractionation and purification of the resulting extract in conventional manners. Examples of the organic solvent to be used are methanol, ethanol, ether, acetone, benzene, toluene and ethyl acetate, but not limited thereto, and therefore other suitable solvents can be also used. Examples of the fractionation and the purification are chromatographies such as silica gel column chromatography, preparative silica gel thin layer chromatography or high performance liquid chromatography, and recrystallization. Eluents to be used herein include ether, hexane, ethyl acetate, benzene, toluene, acetone, methanol, chloroform or dichloromethane, and they can be used alone or in admixture.

Aragusterol C obtained above or known aragusterol A is chemically modified to give other sterol compounds of the present invention.

Namely, aragusterol A is reacted with a halogenating reagent (e.g. $Li_2CuCl_4$ when X is a chlorine atom, or $Li_2CuBr_4$ when X is a bromine atom) to give a sterol compound of the present invention wherein $R^1 = R^2 = H$ and A = -CO-.

Further, a compound of the present invention wherein $R^1$ and/or $R^2$ is an alkanoyl group can be prepared by reacting the compound obtained above with an acid hydride of $R^2O$ (wherein R is an alkanoyl group defined for $R^1$ or $R^2$) or an acylating agent such as an acid halide of RX' (wherein R is as defined above, and X' is a halogen atom) in a solvent such as pyridine in the absence or presence of a base such as 4-dimethylaminopyridine or triethylamine, or reacting with a carboxylic acid of ROH (wherein R is as defined above) in a dehydrating agent such as 1,3-dicyclohexylcarbodiimide or 1,1'-carbonyldiimidazole. As an example of the reaction, aragusterol C is acetylated with acetic anhydride/pyridine to give a sterol compound of the present invention wherein $R^1$ and/or $R^2$ is an acetyl group.

Furthermore, a sterol compound of the present invention wherein A is -CH(OH)- can be prepared by reacting the sterol compound of the present invention wherein A is -CO- with a reducing agent such as sodium cyanoborohydride in a lower alcohol such as methanol or ethanol in the absence or presence of an acid such as acetic acid.

For the use as medicines, the sterol compounds of the present invention can be formulated as oral or parenteral dosage forms, examples of which are tablets, granules, powders, capsules, syrups, suspensions and injections, and they can be suitably chosen depending on the symptom, age and objects of the treatment of the patient. The dosage for adult human treatment is 1 to 500 mg per day, in 2 or 3 divided doses, and can be varied depending on the age, body weight and symptoms of the patient.

Industrial Applicability

According to the present invention, a novel sterol compound (aragusterol C) is isolated from sponges of the genus Xestospongia, and other novel sterol compounds are prepared by chemical modification of the above compound. Furthermore, known aragusterol A is modified chemically to give aragusterol C and novel sterol compounds. These compounds have more excellent antitumor activity than aragusterol A, and therefore are useful as medicines.

Best Mode For Carrying Out The Invention

The present invention is illustrated in more detail by the following examples and an experiment.

Example 1

Sponges of the genus Xestospongia were collected at coral reefs in the sea near Iriomote-jima, Okinawa, Japan and immediately frozen with dry ice. The frozen material (3 kg) was impregnated with methanol (6 ℓ) under cooling. The methanol extract was collected by filtration, and the filtrate was concentrated under reduced pressure to give a residue. The methanol extractions were repeated twice in a similar manner, and the resulting second extract was combined with the first extract. The remaining frozen material was extracted similarly, thereby giving the methanol extracts in the total amount of 2604 g. 131 g of the methanol extract was suspended in 1.5 ℓ of water, and extracted twice with 1.5 ℓ of ethyl acetate. The combined ethyl acetate-soluble product was dried over anhydrous magnesium sulfate and concentrated under reduced pressure to give a residue (12 g). The remaining methanol extract was divided into 11 parts, and each part was partitioned similarly between water and ethyl acetate to give an ethyl acetate-soluble product, which was then combined with the earlier ethyl acetate-soluble product (the total amount of 267 g). The ethyl acetate-soluble product (51.3 g) was subjected to column chromatography on silica gel (Fuji-Davison BW-820MH, 500 g) to give three fractions (Fraction 1 eluted with 3000 ml of hexane : ethyl acetate =5:1, Fraction 2 eluted with 1000 ml of hexane : ethyl acetate =1:1, and Fraction 3 eluted with 1000 ml of ethyl acetate and then with 1000 ml of methanol). The remaining ethyl acetate-soluble product was divided into 4 parts, and each part was separated similarly to give 34.4 g of Fraction 1, 67.8 g of Fraction 2 and 138.4 g of Fraction 3, in total, respectively.

Fraction 2 (18.6 g) was subjected to column chromatography on activated carbon (60 g) to give 3 fractions (Fraction 2-1 eluted with 1000 ml of methanol, Fraction 2-2 eluted with 1000 ml of ethyl acetate and Fraction 2-3 eluted with 2000 ml of chloroform). The remaining Fraction 2 was divided into 4 parts, and each part was separated similarly to give 14.8 g of Fraction 2-1, 25.6 g of Fraction 2-2, and 27.3 g of Fraction 2-3, in total, respectively.

Fraction 2-3 (4.3 g) was subjected to column chromatography on silica gel (Fuji-Davison BW-820MH, 100 g) to give 7 fractions (Fraction 2-3-1 eluted with 200 ml of hexane : ethyl acetate =2:1; Fraction 2-3-2 eluted with 150 ml of hexane : ethyl acetate =2:1; Fraction 2-3-3 eluted with 200 ml of hexane : ethyl acetate =2:1; Fraction 2-3-4 eluted with 150 ml of hexane : ethyl acetate =2:1; Fraction 2-3-5 eluted with 650 ml of hexane : ethyl acetate =2:1; Fraction 2-3-6 eluted with 700 ml of hexane : ethyl acetate =2:1; and Fraction 2-3-7 eluted with 300 ml of methanol). The remaining Fraction 2-3 was divided into 5 parts, and each part was separated in a similar manner.

The combined Fraction 2-3-5 (10.52 g) was subjected to flash chromatography (Fuji-Davison BW-300, 300 g) to give 7 fractions (Fraction 2-3-5-1 eluted with 1100 ml of hexane : acetone =4:1; Fraction 2-3-5-2 eluted with 500 ml of hexane : acetone =4:1; Fraction 2-3-5-3 eluted with 1000 ml of hexane : acetone =4:1; Fraction 2-3-5-4 eluted with 400 ml of hexane : acetone =4:1; Fraction 2-3-5-5 eluted with 800 ml of hexane : acetone =4:1; Fraction 2-3-5-6 eluted with 400 ml of hexane : acetone =4:1; and Fraction 2-3-5-7 eluted with 500 ml of ethyl acetate).

Fraction 2-3-6 (2.9 g) was similarly subjected to flash chromatography (Fuji-Davison BW 300, 100 g) eluting with hexane : acetone =4:1 to give 8 fractions (Fraction 2-3-6-1 ~ 8).

Fractions 2-3-5-6, 2-3-6-3 and 2-3-6-4 were combined together (2.69 g) and purified by flash chromatography (Fuji-Davison BW-300, 200 g) eluting with hexane : acetone = 3:1, and the resulting crystalline fraction (2.20 g) was recrystallized from hexane - ethyl acetate to give aragusterol C (852 mg) as colorless pillars.

m.p. 204 ~ 205°C.

| Elementary analysis: | | | |
|---|---|---|---|
| Calcd. (%) | C:70.35, | H:9.57, | Cl:7.16 |
| Found (%) | C:70.32, | H:9.65, | Cl:7.25 |

IR(KBr) $cm^{-1}$;

3400, 3266, 1693

$^1$H-NMR (400 MHz, $CDCl_3$) δ (ppm);

0.18 (1H, q, J=4.5Hz), 0.27 (2H, m), 0.53 (1H, m), 0.98 (6H, s), 1.02 (3H, d, J=5.4Hz), 1.03 (3H, s), 2.03 (1H, ddd, J=2.1Hz, 6.4Hz, 13.1Hz), 2.09 (1H, ddd, J=1.7Hz, 38Hz, 15.0Hz), 2.38 (1H, dt, J=6.4Hz, 13.5Hz), 3.44 (1H, dd, J=4.5Hz, 11.1Hz), 3.88 (2H, s), 3.97 (1H, dd, J=3.3Hz, 11.1Hz)

$^{13}$C-NMR (100 MHz, $CDCl_3$) δ (ppm);

9.0 ($CH_3$), 11.5 ($CH_3$), 12.3 (CH), 12.6 ($CH_2$), 18.9 ($CH_3$), 19.3 ($CH_3$), 23.5 ($CH_2$), 23.6 ($CH_2$), 28.0 (CH), 28.9 ($CH_2$), 29.8 ($CH_2$), 31.1 ($CH_2$), 33.9 (CH), 35.3 (CH), 35.7 (C), 37.8 ($CH_2$), 38.1 ($CH_2$), 38.6 ($CH_2$), 44.6 ($CH_2$), 46.6 (CH), 47.4 ($CH_2$), 49.2 (C), 52.5 (CH), 54.1 (CH), 55.0 (CH), 71.4 (CH), 77.1 (C), 77.8 (CH), 211.6 (CO)

FABMS m/z;

495 ($MH^+$), 497 [$(MH+2)^+$]

$[\alpha]_D^{26}$; +20.1° (c 0.35, $CHCl_3$)

Example 2

To a solution of aragusterol C (101 mg) in pyridine (1 ml) was added acetic anhydride (1 ml), followed by stirring at room temperature for 71 hours. The reaction solution was diluted with ether (20 ml) and washed with water (3 ml). The ether solution was successively washed with a saturated aqueous copper sulfate solution (3×2 ml), water (3 ml), a saturated aqueous sodium bicarbonate solution (2×10 ml), water (3 ml) and a saturated aqueous sodium chloride solution (3 ml). The ether solution was dried over anhydrous sodium sulfate, and after evaporation of the solvent under reduced pressure, the resulting crude product (135 mg) was subjected to column chromatography on silica gel (Merck, Silica gel 60, 15 g) eluting with hexane : acetone =4:1 (13 ml/fraction), and aragusterol C diacetate (113 mg) was obtained from the fractions 7 ~ 14.

m.p. 102 ~ 103°C.

IR (KBr) $cm^{-1}$;

3490, 1736, 1718, 1250

$^1$H-NMR (400 MHz, $CDCl_3$) δ (ppm);

0.15 (1H, m), 0.23 (2H, m), 0.40 (1H, m), 0.53 (1H, m), 0.99 (3H, d, J=6.7Hz), 1.00 (3H, d, J=6.3Hz), 1.01 (3H, s), 1.07 (3H, s), 2.04 (3H, s), 2.06 (3H, s), 2.11 (1H, ddd, J=1.9Hz, 4.0Hz, 15.1Hz), 2.25 (1H, dd, J=13.9Hz, 15.1Hz), 2.35 (1H, dt, J=6.3Hz, 13.3Hz), 2.80 (1H, s), 3.76 (1H, d, J=11.5Hz), 3.83 (1H, d, J=11.5Hz), 4.68 (1H, dd, J=4.8Hz, 11.1Hz), 5.26 (1H, br d, J=11.0Hz)

$^{13}$C-NMR (100 MHz, $CDCl_3$) δ (ppm);

10.3 ($CH_3$), 11.4 ($CH_3$), 12.4 (CH), 12.6 ($CH_2$), 18.7 ($CH_3$), 19.1 ($CH_3$), 21.1 ($CH_3$), 21.7 ($CH_3$), 23.4 ($CH_2$), 23.9 ($CH_2$), 27.5 ($CH_2$), 27.9 (CH), 28.8 ($CH_2$), 30.9 ($CH_2$), 33.9 (CH), 35.1 (CH), 35.7 (C), 36.9 ($CH_2$), 37.9 ($CH_2$), 38.4 ($CH_2$), 44.5 ($CH_2$), 46.4 (CH), 47.5 (C), 47.6 ($CH_2$), 52.1 (CH), 54.5 (CH), 56.6 (CH), 75.4 (CH), 76.8 (C), 80.0 (CH), 170.2 (CO), 170.8 (CO), 211.1 (CO)

FABMS m/z;

579 ($MH^+$), 581 [$(MH+2)^+$]

Example 3

To a solution of aragusterol A (66 mg) in tetrahydrofuran (1.5 ml) was added under an argon atmosphere a solution of $Li_2CuCl_4$ prepared by the method described in the literature (Tetrahedron Letters, vol. 27, page 3697, 1986) in tetrahydrofuran (1 mol/ℓ, 0.29 ml), followed by stirring at room temperature for 1.5 hours. Ethyl acetate was added to the reaction solution, and the mixture was successively washed with water and a saturated aqueous sodium chloride solution and dried over anhydrous magnesium sulfate. After evaporation of the solvent under reduced pressure, the resulting crude product was subjected to column chromatography on silica gel eluting with dichloromethane : acetone =6:1 to give aragusterol C (69 mg), of which physical values (melting point, IR, $^1$H-NMR and $^{13}$C-NMR) were identical with those of aragusterol C derived from the sponges.

Example 4

To a solution of aragusterol A (30 mg) in tetrahydrofuran (600 μl) was added a solution of $Li_2CuBr_4$ prepared by a method described in the literature cited in Example 3 in 1M tetrahydrofuran (300 μl) under an argon atmosphere at room temperature, followed by stirring for 30 minutes. The reaction solution was diluted with ether, washed with water and a saturated aqueous sodium chloride solution, and dried over anhydrous magnesium sulfate. Evaporation of the solvent under reduced pressure gave a compound of the present invention wherein $R^1$ and $R^2$ are each a hydrogen atom, X is a bromine atom and A is -CO- (18 mg) as a colorless powder.

$^1$H-NMR (300 MHz, $CDCl_3$) δ (ppm);

0.16 (1H, m), 0.24 (2H, m), 0.51 (1H, m), 0.96 (3H, s), 1.01 (3H, s), 1.01 (1H, d, J=5.5Hz), 1.02 (3H, s), 3.44 (1H, dd, J=4.6Hz, 7.15Hz), 3.74 (1H, d, J=11.0Hz), 3.78 (1H, d, J=11.0Hz), 3.97 (1H, br d, J=10.6Hz)

SIMS (+KI) m/z;

577 ($MK^+$), 579 [$(MK+2)^+$]

Example 5

To a solution of aragusterol C (25 mg) in methanol (1 ml) was added acetic acid (40 μl). The solution was cooled to 0°C, and sodium cyanoborohydride (20 mg) was added thereto with stirring, followed by further stirring at 0°C for 3 hours. The reaction solution was diluted with ether, washed with water and a saturated aqueous sodium chloride solution, and dried over anhydrous magnesium sulfate. The solvent was evaporated under reduced pressure, and the resulting crude product was recrystallized from ethyl acetatehexane to give a compound wherein $R^1$ and $R^2$ are each a hydrogen atom, X is a chlorine atom and A is -CH(β-OH)-(19 mg) as a colorless powder.

m.p. 115 ~ 118°C.

IR (KBr) $cm^{-1}$;

3381

$^1$H-NMR (300 MHz, $CDCl_3$) δ (ppm);

0.16 (1H, m), 0.24 (2H, m), 0.47 (1H, m), 0.69 (3H, s), 0.79 (3H, s), 0.94 (3H, s), 1.01 (1H, d, J=6.0Hz), 1.92 (1H, m), 2.13 (1H, t, J=9.2Hz), 2.91 (1H, d, J=4.1Hz), 3.07 (1H, d, J=4.1Hz), 3.36 (1H, dd, J=4.6Hz, 11.0Hz), 3.46 (1H, dd, J=2.7Hz, 11.2Hz), 3.58 (1H, m)

SIMS (+KI) m/z;

535 ($MK^+$), 537 [$(MK+2)^+$]

$[\alpha]_D^{28}$; +3.75° (c 0.16, $CHCl_3$)

Example 6

The compound wherein $R^1$ and $R^2$ are each a hydrogen atom, X is a bromine atom and A is -CO-obtained in Example 4 was treated by a method similar to that of Example 5 to give a compound wherein $R^1$ and $R^2$ are each a hydrogen atom, X is a bromine atom and A is -CH(β-OH)- as a colorless powder.

$^1$H-NMR (300 MHz, $CDCl_3$) δ (ppm);

0.16 (1H, m), 0.24 (2H, m), 0.51 (1H, m), 0.82 (3H, s), 0.94 (3H, s), 0.97 (3H, s), 1.01 (1H, d, J=5.9Hz), 3.43 (1H, dd, J=4.6Hz, 11.3 Hz), 3.59 (1H, m), 3.73 (1H, d, J=10.8Hz), 3.79 (1H, d, J=10.8Hz), 3.96 (1H, br d, J=10.6Hz)

SIMS (+KI) m/z;

579 ($MK^+$), 581 [$(MK+2)^+$]

Experiment: In Vivo Life-Prolonging Effect against L1210 Leukemia

L1210 leukemia cells were intraperitoneally transplanted into DBA/2 female mice at 1 x $10^5$ cells per animal. Tumor cells were collected from ascites of the animal on day 6 or 7 after the transplantation. A cell suspension containing 5 x $10^5$ viable cells/ml in Hank's balanced salt solution was prepared, and 0.2 ml of the cell solution was transplanted intraperitoneally into each of $CDF_1$ female mice (8 weeks old) (1 x $10^5$ cells/animal). The transplantation day is regarded as day 0. Each of suspensions of aragusterol C and aragusterol A in 0.5 % of gum arabic/saline solution was administered intraperitoneally for 5 days after the transplantation day.

The effect was evaluated by T/C % calculated according to the following equation using median survival time (MST) of the mouse. T/C(%) = (MST of the group treated by drug/MST of the control group) × 100

Results are shown in Table 1.

Table 1

| Test drug | dose (mg/kg) | T/C (%) | Body weight gain (g) |
|---|---|---|---|
| aragusterol C | 1.6 | 257 | -0.1 |
| | 6.25 | 181 | -0.3 |
| aragusterol A | 1.6 | 220 | 0.3 |
| | 6.25 | 124 | -0.5 |
| (Note) Body weight gain = (Body weight on day 4) - (Body weight on day 1) | | | |

## Claims

1. A sterol compound represented by the following formula:

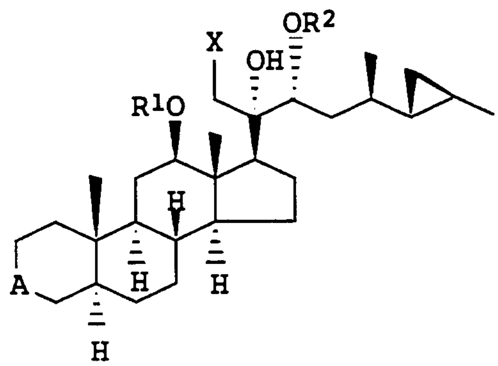

wherein X is a halogen atom, $R^1$ and $R^2$ are the same or different, and are each a hydrogen atom or an alkanoyl group having 2 to 5 carbon atoms, and A is -CO- or -CH(OH)-.

2. A sterol compound represented by the following formula:

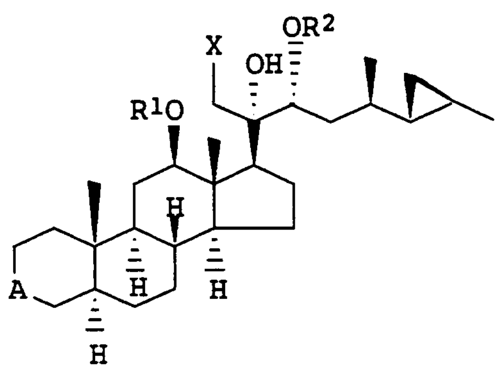

wherein X is a halogen atom, $R^1$ and $R^2$ are the same or different, and are each a hydrogen atom or an acetyl group, and A is -CO- or -CH(OH)-.

## INTERNATIONAL SEARCH REPORT

International application No.
PCT/JP94/01044

**A. CLASSIFICATION OF SUBJECT MATTER**

Int. $Cl^5$ C07J9/00, A61K31/575, A61K31/56

According to International Patent Classification (IPC) or to both national classification and IPC

**B. FIELDS SEARCHED**

Minimum documentation searched (classification system followed by classification symbols)

Int. $Cl^5$ C07J9/00, A61K31/575, A61K31/56

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| P | JP, A, 5-4998 (TAISHO PHARMACEUT KK), January 14, 1993 (13. 01. 93) & EP, A, 467664 & US, A, 5122521 | 1-2 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| August 23, 1994 (23. 08. 94) | September 13, 1994 (13. 09. 94) |
| Name and mailing address of the ISA/ Japanese Patent Office | Authorized officer |
| Facsimile No. | Telephone No. |

Form PCT/ISA/210 (second sheet) (July 1992)